# EUROPEAN PATENT APPLICATION

(11) **EP 4 772 651 A1**
(43) Date of publication of application: **08.07.2026**
(21) Application number: 25382005.4
(22) Date of filing: 07.01.2025
(51) Int. Cl.: C12Q 1/6886

(54) **METHOD FOR PREDICTING PROGRESSION OF PROSTATE CANCER**

(71) Applicant: Fundación para la Investigación Biomédica del Hospital Universitario 12 de Octubre, 28041 Madrid (ES); Universidad de Murcia, 30003 Murcia (ES); Fundación MD Anderson International España, 28033 Madrid (ES)
(72) Inventor: GONZÁLEZ BILLALABEITIA, Enrique, 28041 Madrid (ES); PÉREZ NAVARRO, Enrique, 28041 Madrid (ES); BOTÍA BLAYA, Juan Antonio, 30003 Murcia (ES); PALMA MÉNDEZ, José Tomás, 30003 Murcia (ES); GRANDE PULIDO, Enrique, 28033 Madrid (ES)
(74) Representative: Hoffmann Eitle

(57) **Abstract**

The present invention relates to methods for predicting the progression of prostate cancer, specifically metastatic castration-resistant prostate cancer (mCRPC), in an individual. The invention employs gene expression profiling combined with predictive modeling to assess the likelihood of overall survival and biochemical recurrence following treatment.

## Description

### Field of the Invention

The present invention relates to methods for predicting the progression of prostate cancer, specifically metastatic castration-resistant prostate cancer (mCRPC), in an individual. The invention employs gene expression profiling combined with predictive modeling to assess the likelihood of overall survival and biochemical recurrence following treatment.

### Background of the Invention

Prostate cancer is a leading cause of cancer-related mortality among men. Metastatic castration-resistant prostate cancer (mCRPC) represents an advanced stage of the disease characterized by poor prognosis and limited treatment options. Accurate prediction of disease progression is critical for guiding therapeutic strategies and improving patient outcomes.

Currently, clinical assessments such as PSA levels and imaging studies are commonly used to monitor prostate cancer progression; however, they lack precision in predicting overall survival and recurrence. Despite the availability of various genomic panels and clinical metrics, there remains a significant unmet need for a robust and reproducible molecular signature that can accurately predict progression and overall survival after mCRPC therapies such as enzalutamide. Classic tests often fail due to low predictive power, irreproducibility across cohorts, and unsatisfactory inclusion of changes in gene expression over time. Advances in gene expression profiling have revealed molecular signatures that are associated with prostate cancer behavior, offering a novel approach to prognostic assessment.

The present invention addresses these challenges by providing a method to predict prostate cancer progression using a predictive model based on expression levels of a collection of signature genes derived from a biological sample of the individual.

### Summary of the invention

An initial aspect of the invention refers to a method for predicting progression of prostate cancer in an individual, the method comprising:
(a) receiving expression levels of a collection of signature genes from a biological sample taken from said individual, wherein said collection of signature genes comprises at least 2, 3, 4, 5, 6, 7, 8, 9 ... 22 genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8;
(b) applying the expression levels to a predictive model relating expression levels of said collection of signature genes with prostate cancer progression; and
(c) evaluating an output of said predictive model to predict progression of prostate cancer in said individual.

In an embodiment, the method comprises:
(a) receiving expression levels of a collection of signature genes from a biological sample taken from said individual, wherein said collection of signature genes comprises at least all genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8, and
(b) applying the expression levels to a predictive model relating expression levels of said collection of signature genes with prostate cancer progression; and
(c) evaluating an output of said predictive model to predict progression of prostate cancer in said individual.

In another embodiment, the prostate cancer is metastatic castration-resistant prostate cancer (mCRPC).

In another embodiment, said output of the predictive model predicts a likelihood of clinical recurrence of prostate cancer in the individual after said individual has undergone treatment for prostate cancer, wherein said treatment is enzalutamide.

In another embodiment, said output of the predictive model predicts a likelihood of biochemical recurrence of prostate cancer in the individual after said individual has undergone treatment for prostate cancer.

In another embodiment, said output of the predictive model predicts a likelihood of overall survival of the individual after said individual has undergone treatment for prostate cancer.

In another embodiment, the method further comprises combining the gene expression levels of said signature genes with one or more other biomarkers to predict progression of prostate cancer in said individual such as one or more other biomarkers selected from the group consisting of germline mutations, somatic mutations, DNA methylation markers, protein markers, and any combinations thereof.

In another embodiment, the expression levels of a collection of signature genes comprise gene expression levels measured at multiple times.

In another embodiment, the biological sample is a biological fluid selected from the group consisting of blood, preferably whole blood.

A further aspect of the invention refers to a system for predicting progression of prostate cancer in an individual, the system comprising:
an apparatus configured to determine expression levels of nucleic acids from a biological sample taken from the individual; and
hardware logic designed or configured to perform operations comprising:
   (a) receiving expression levels of a collection of signature genes from a biological sample taken from said individual, wherein said collection of signature genes comprises at least two genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8;
   (b) applying the expression levels to a predictive model relating expression levels of said collection of signature genes with prostate cancer progression; and
   (c) evaluating an output of said predictive model to predict progression of prostate cancer in said individual.

In an embodiment, said collection of signature genes comprises at least all genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8; wherein the prostate cancer is metastatic castration-resistant prostate cancer (mCRPC), and wherein said output of the predictive model predicts a likelihood of clinical recurrence of prostate cancer in the individual after said individual has undergone treatment for prostate cancer, wherein said treatment is enzalutamide.

In another embodiment, the biological sample is a body fluid selected from the group consisting of blood, preferably whole blood.

A still further aspect of the invention refers to a method comprising:
- determining a gene expression level for the signature genes: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8, to obtain a subject expression profile for a subject,
and further comprising:
- classifying the subject as having a good prognosis or a poor prognosis of prostate cancer based on the subject expression profile, wherein the good prognosis predicts an increased likelihood of survival within a predetermined period after initial diagnosis and/or no progression of disease after primary treatment, and the poor prognosis predicts an aggressive disease, a decreased likelihood of survival, and an increased likelihood of biochemical recurrence, clinical recurrence, and/or the presence of local or distant metastases, within a predetermined period after initial diagnosis; and/or
- classifying the subject as having or not having a predisposition of prostate cancer that is susceptible to disease progression based on the subject expression profile, wherein the predisposition predicts an aggressive disease, decreased likelihood of survival, and an increased likelihood of biochemical recurrence, clinical recurrence, and/or the presence of local or distant metastases, within a predetermined period after initial diagnosis.

In an embodiment, the prostate cancer is metastatic castration-resistant prostate cancer (mCRPC), and said individual has undergone treatment for prostate cancer, wherein preferably said treatment is enzalutamide.

A final aspect refers to a composition comprising enzalutamide for use in the treatment of metastatic castration-resistant prostate cancer (mCRPC) in an individual having said disease and classified as having good prognosis in accordance with the previous aspect of the invention.

### Description of embodiments of the invention

### Definitions

Unless otherwise indicated, the practice of the method and system disclosed herein involves conventional techniques and apparatus commonly used in molecular biology, microbiology, protein purification, protein engineering, protein and DNA sequencing, and recombinant DNA fields, which are within the skill of the art. Such techniques and apparatus are known to those of skill in the art and are described in numerous texts and reference works (See e.g., Sambrook et al, "Molecular Cloning: A Laboratory Manual," Third Edition (Cold Spring Harbor), [2001]); and Ausubel et al, "Current Protocols in Molecular Biology" [1987]).

Numeric ranges are inclusive of the numbers defining the range. It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Unless defined otherwise herein, all technical and scientific terms used herein have the same meaning as commonly understood by one of ordinary skill in the art. Various scientific dictionaries that include the terms included herein are well known and available to those in the art. Although any methods and materials similar or equivalent to those described herein find use in the practice or testing of the embodiments disclosed herein, some methods and materials are described.

The terms defined immediately below are more fully described by reference to the Specification as a whole. It is to be understood that this disclosure is not limited to the particular methodology, protocols, and reagents described, as these may vary, depending upon the context they are used by those of skill in the art.

The headings provided herein are not intended to limit the disclosure.

As used herein, the singular terms "a," "an," and "the" include the plural reference unless the context clearly indicates otherwise.

"Nucleic acid sequence," "expressed nucleic acid," or grammatical equivalents thereof used in the context of a corresponding signature gene means a nucleic acid sequence whose amount is measured as an indication of the gene's expression level. The nucleic sequence can be a portion of a gene, a regulatory sequence, genomic DNA, cDNA, RNA including mRNA and rRNA, or others. A preferred embodiment utilizes mRNA as the primary target sequence. As is outlined herein, the nucleic acid sequence can be a sequence from a sample, or a secondary target such as, for example, a product of a reaction such as a detection sequence from an invasive cleavage reaction, a ligated probe from an OLA or DASL (cDNA- mediated Annealing, Selection, and Ligation) reaction, an extended probe from a PCR reaction, or PCR amplification product (e.g., "amplicon"). A nucleic acid sequence corresponding to a signature gene can be any length, with the understanding that longer sequences are more specific. Probes are made to hybridize to nucleic acid sequences to determine the presence or absence of expression of a signature gene in a sample.

"Prostate cancer" as used herein includes carcinomas, including, carcinoma in situ, invasive carcinoma, metastatic carcinoma and pre-malignant conditions.

As used herein the term "comprising" means that the named elements are included, but other element (e.g., unnamed signature genes) may be added and still represent a composition or method within the scope of the claim. The transitional phrase "consisting essentially of means that the associated composition or method encompasses additional elements, including, for example, additional signature genes, that do not affect the basic and novel characteristics of the disclosure.

As used herein, the term "signature gene" refers to a gene whose expression is correlated, either positively or negatively, with disease extent or outcome or with another predictor of disease extent or outcome. In some embodiments, a gene expression score (GEX) can be statistically derived from the expression levels of a set of signature genes and used to diagnose a condition or to predict clinical course. In some embodiments, the expression levels of the signature gene may be used to predict progression of PCa without relying on a GEX. A "signature nucleic acid" is a nucleic acid comprising or corresponding to, in case of cDNA, the complete or partial sequence of a RNA transcript encoded by a signature gene, or the complement of such complete or partial sequence. A signature protein is encoded by or corresponding to a signature gene of the disclosure.

The term "relapse prediction" is used herein to refer to the prediction of the likelihood of cancer recurrence in patients with no apparent residual tumor tissue after treatment. The predictive methods of the present disclosure can be used clinically to make treatment decisions by choosing the most appropriate treatment modalities for any particular patient. The predictive methods of the present disclosure also can provide valuable tools in predicting if a patient is likely to respond favorably to a treatment regimen, such as surgical intervention, chemotherapy with a given drug or drug combination, and/or radiation therapy.

The Gleason grading system is based on the glandular pattern of the tumor. Gleason grade takes into account the ability of the tumor to form glands. A pathologist, using relatively low magnification, performs the histologic review necessary for assigning the Gleason grade. The range of grades is 1-5: 1, 2 and 3 are considered to be low to moderate in grade; 4 and 5 are considered to be high grade. The prognosis for a given patient generally falls somewhere between that predicted by the primary grade and a secondary grade given to the second most prominent glandular pattern. When the two grades are added the resulting number is referred to as the "Gleason score". The Gleason Score is a more accurate predictor of outcome than either of the individual grades. Thus, the traditionally reported Gleason score will be the sum of two numbers between 1-5 with a total score from 2-10. It is unusual for the primary and secondary Gleason grade to differ by more than one, such that the only way that there can be a Gleason score 7 tumor is if the primary or secondary Gleason grade is 4. Because of the presence of grade 4 glandular patterns in tissue having Gleason score 7, these tumors can behave in a much more aggressive fashion than those having Gleason score 6. In a recent study of over 300 patients, the disease specific survival for Gleason score 7 patients was 10 years. In contrast, Gleason score 6 patients survived 16 years and Gleason 4-5 for 20 years. It is therefore clear that the prognosis for men with Gleason score 7 tumors is worse than for men with Gleason score 5 and 6 tumors. Under certain circumstances it is suggested that men with Gleason 7 tumors can be considered for clinical trials.

The terms "polynucleotide," "nucleic acid" and "nucleic acid molecules" are used interchangeably and refer to a covalently linked sequence of nucleotides (i.e., ribonucleotides for RNA and deoxyribonucleotides for DNA) in which the 3 ' position of the pentose of one nucleotide is joined by a phosphodiester group to the 5' position of the pentose of the next. The nucleotides include sequences of any form of nucleic acid, including, but not limited to RNA and DNA molecules. The term "polynucleotide" includes, without limitation, single- and double-stranded polynucleotide.

The term "Next Generation Sequencing (NGS)" herein refers to sequencing methods that allow for massively parallel sequencing of clonally amplified molecules and of single nucleic acid molecules. Non-limiting examples of NGS include sequencing-by-synthesis using reversible dye terminators, and sequencing-by-ligation.

The term "read" refers to a sequence read from a portion of a nucleic acid sample. Typically, though not necessarily, a read represents a short sequence of contiguous base pairs in the sample. The read may be represented symbolically by the base pair sequence (in ATCG) of the sample portion. It may be stored in a memory device and processed as appropriate to determine whether it matches a reference sequence or meets other criteria. A read may be obtained directly from a sequencing apparatus or indirectly from stored sequence information concerning the sample. In some cases, a read is a DNA sequence of sufficient length (e.g., at least about 25 bp) that can be used to identify a larger sequence or region, e.g., that can be aligned and specifically assigned to a chromosome or genomic region or gene.

As used herein, the terms "aligned," "alignment," or "aligning" refer to the process of comparing a read or tag to a reference sequence and thereby determining whether the reference sequence contains the read sequence. If the reference sequence contains the read, the read may be mapped to the reference sequence or, in certain embodiments, to a particular location in the reference sequence. In some cases, alignment simply tells whether or not a read is a member of a particular reference sequence (i.e., whether the read is present or absent in the reference sequence). For example, the alignment of a read to the reference sequence for human chromosome 13 will tell whether the read is present in the reference sequence for chromosome 13. A tool that provides this information may be called a set membership tester. In some cases, an alignment additionally indicates a location in the reference sequence where the read or tag maps to. For example, if the reference sequence is the whole human genome sequence, an alignment may indicate that a read is present on chromosome 13, and may further indicate that the read is on a particular strand and/or site of chromosome 13.

Aligned reads or tags are one or more sequences that are identified as a match in terms of the order of their nucleic acid molecules to a known sequence from a reference genome. Alignment can be done manually, although it is typically implemented by a computer algorithm, as it would be impossible to align reads in a reasonable time period for implementing the methods disclosed herein. One example of an algorithm from aligning sequences is the Efficient Local Alignment of Nucleotide Data (ELAND) computer program distributed as part of the Illumina Genomics Analysis pipeline. Alternatively, a Bloom filter or similar set membership tester may be employed to align reads to reference genomes. See US Patent Application No. 61/552,374 filed October 27, 2011 which is incorporated herein by reference in its entirety. The matching of a sequence read in aligning can be a 100% sequence match or less than 100% (non-perfect match).

The term "mapping" used herein refers to specifically assigning a sequence read to a larger sequence, e.g., a reference genome, by alignment.

The term "subject" herein refers to a human subject as well as a non- human subject such as a mammal, an invertebrate, a vertebrate, a fungus, a yeast, a bacterium, and a virus. Although the examples herein concern humans and the language is primarily directed to human concerns, the concepts disclosed herein are applicable to genomes from any plant or animal, and are useful in the fields of veterinary medicine, animal sciences, research laboratories and such.

The term "condition" herein refers to "medical condition" as a broad term that includes all diseases and disorders, but can include [injuries] and normal health situations, such as pregnancy, that might affect a person's health, benefit from medical assistance, or have implications for medical treatments.

The term "sensitivity" as used herein is equal to the number of true positives divided by the sum of true positives and false negatives.

The term "specificity" as used herein is equal to the number of true negatives divided by the sum of true negatives and false positives.

This disclosure provides improved predictive models that incorporate tumor biomarkers and that better distinguish between indolent cases and metastatic disease. Some embodiments provide a method for predicting progression of prostate cancer, preferably metastatic castration-resistant prostate cancer (mCRPC), in an individual, the method comprising: (a) receiving expression levels of a collection of signature genes from a biological sample taken from said individual, wherein said collection of signature genes comprises at least two or more genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8; (b) applying the expression levels to a predictive model relating expression levels of said collection of signature genes with prostate cancer progression; and (c) evaluating an output of said predictive model to predict progression of prostate cancer in said individual.

In some embodiments, the output of the predictive model predicts a likelihood of overall survival or clinical recurrence of prostate cancer in the individual after said individual has undergone treatment for prostate cancer, wherein the prostate cancer is preferably metastatic castration-resistant prostate cancer (mCRPC), and said treatment is enzalutamide.

In some embodiments, the 22 markers of the panel are differentially expressed/regulated between PCa cases with and without recurrence, and are predictive of aggressive disease. In some embodiments, the predictive models include these 22 markers along with pre-operative PSA levels, Gleason score, and age at diagnosis, which models show greater prediction than models having clinical variables alone. One skilled in the art understands that further validation of the models using additional datasets will allow improvement of the predictive power of the models, which may include different coefficients of the models. In some embodiments, one or more genes can be selected from the panel to form predictive models for evaluation of PCa progression.

### Identifying Gene Expression Panel and Developing Predictive Model

Some embodiments the disclosure provides methods for developing predictive models for determining PCa progression. In some embodiments, the models are developed using data collected from patients known to have prostate cancer. In some embodiments, the predictive models describe the correlation between expression levels of signature genes measured in whole blood and clinical recurrence of PCa or overall survival in patients providing the biological samples. In various embodiments, the disclosure provides a panel of 22 signature genes that correlate with overall survival or PCa recurrence in the prostate cancer patients. In some embodiments, the disclosure further provides methods to predict PCa development, recurrence, and/or overall survival for an individual using the individual's expression levels of one or more of the signature genes. In some embodiments, the predictive model includes the expression levels of at least one gene that is selected from the group including: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8.

In some embodiments, the gene expression data may be preprocessed by normalization, background correction, and/or batch effect correction. The pre- processed data may then be analyzed for differential expression of genes for no evidence of disease (NED) group versus clinical recurrence (CR) group.

In some embodiments, one or more of the following genes: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8, may be used in a predictive model. In some embodiments, the one or more genes may be selected by their correlation with recurrence or overall survival in the training data set to develop the predictive models. In some embodiments, the one or more genes may be selected by their reliability ranks. In some embodiments, the panel of signature genes include at least 1, 2, 3, 4, 5, 6, 7, 8, 9, or 10 of SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8. In some embodiments, the one or more genes may be selected by their predictive power rankings.

In some embodiments, various clinical variables (e.g., PSA level, Gleason score, operation year and age) will be included in the same logistic model along with the signature genes. CoefFicients will be defined for each variable (gene expression and clinical values). This logistic regression model will provide a probability of having a clinical recurrence given the provided gene expression scores and clinical variables. This probability will be a number between 0-1, and it will indicate for each given patient the probability of having a clinical recurrence.

In some embodiments, in addition to identifying the coefficients of the predictive model, the disclosure identifies the most useful specificity and sensitivity a user wishes to have for a specific risk probability. Based on the desired specificity and sensitivity levels, the method will report the risk status of each patient. For example, we may find that given the specificity and sensitivity of our model, a patient with 45% chance of clinical recurrence might be better off being classified as high-risk of recurrence rather than low-risk or vice versa. In other words, more user-friendly criteria can be chosen based on more detailed analyses in further datasets to determine the most practical interpretation of the risk probability depending on how much clinicians want to risk having a false positive or a false negative.

One skilled in the art can readily determine other combinations of signature genes sufficient to practice the disclosures claimed herein. For example, based on the stability selection ranking of SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8 or the p-values of the univariate comparison between the NED and the CR groups, one skilled in the art can readily determine a sub-combination of prostate cancer signature genes suitable for methods of the disclosure. Those exemplary genes having lowest stability selection ranks can be excluded, with the remaining genes providing a sufficient collection of isolated prostate cancer signature genes suitable for relapse prediction of prostate cancer. Similarly, genes having the largest p-value may be excluded.

Thus, the disclosure provides a method of predicting prostate cancer relapse or overall survival based on the expression patterns for any subset of the 22 genes SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8 including, for example, at least 1, 2, 3, 4, 5, 6, 7, 8, 9, 10, 11, 12, 13, 14, 15, 16, 17, 18, 19, 20, 21, or 22 genes. In some embodiments, the disclosure also provides a method of predicting prostate cancer progression based on the expression patterns for any subset of the set of genes consisting of SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8.

In general, it is preferable to use signature genes for which the difference between the level of expression of the signature gene in prostate cancer cells or prostate-associated body fluids and the level of expression of the same signature gene in normal prostate cells or prostate-associated body fluids is as great as possible. Although the difference can be as small as the limit of detection of the method for assessing expression of the signature gene, it is preferred that the difference be at least greater than the standard error of the assessment method, and preferably a difference of at least 1.1-, 1.2-, 1.3-, 1.4-, 1.5-, 1.6-, 1.7-, 1.8-, 1.9-, 2-, 3- , 4-, 5-, 6-, 7-, 8-, 9-, 10-, 15-, 20-, 25-, 100-, 500-, 1000-fold or greater.

The disclosure also provides a collection of isolated probes specific for prostate cancer signature genes comprising at least two genes selected from the group consisting of SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8. The disclosure includes compositions, kits, and methods for assessing the probability of relapse or overall survival of cancer for an individual from which a sample is obtained.

The practice of the present disclosure employs, unless otherwise indicated, conventional techniques of molecular biology (including recombinant techniques), microbiology, cell biology, and biochemistry, which are within the skill of the art. Such techniques are explained in the literature, such as, "Molecular Cloning: A Laboratory Manual", Second edition (Sambrook et al, 1989); "Oligonucleotide Synthesis" (M. J. Gait, ed., 1984); "Animal Cell Culture" (R. I. Freshney, ed., 1987); "Methods in Enzymology" (Academic Press, Inc.); "Handbook of Experimental Immunology", Fourth edition (D. M. Weir & C. C. Blackwell, eds., Blackwell Science Inc., 1987); "Gene Transfer Vectors for Mammalian Cells" (J. M. Miller & M. P. Calos, eds., 1987); "Current Protocols in Molecular Biology" (F. M. Ausubel et al, eds., 1987); and "PCR: The Polymerase Chain Reaction", (Mullis et al, eds., 1994).

Although the use of the 28 genes, and subsets thereof, has been exemplified with respect to prognosis and diagnosis methods utilizing expression levels of mRNA species produced by these genes, it will be understood that similar diagnostic and prognostic methods can utilize other measures such as methylation levels for the genes which can be correlated with expression levels or a measure of the level or activities of the protein products of the genes. Methylation can be determined using methods known in the art such as those set forth in US 6,200,756 or US 2003/0170684, each of which is incorporated herein by reference. The level and activity of proteins can be determined using methods known in the art such as antibody detection techniques or enzymatic assays particular to the activity being evaluated. Furthermore, prognosis or diagnosis can be based on the presence of mutations or polymorphisms identified in the genes that affect expression of the gene or activity of the protein product.

Information relevant to the patient's diagnosis include, but are not limited to, age, ethnicity, serum PSA at the time of surgery, tumor localization, pertinent past medical history related to co-morbidity, other oncological history, family history for cancer, physical exam findings, radiological findings, biopsy date, biopsy result, types of operation performed (radical retropubic or radical perineal prostatectomy), TNM staging, neoadjuvant therapy (i.e. chemotherapy, hormones), adjuvant or salvage radiotherapy, hormonal therapy for a rising PSA (biochemical disease relapse), local vs. distant disease recurrence and survival outcome. These clinical variables may be included in the predictive model in various embodiments.

In some embodiments, suitable biological samples include, but are not limited to, circulating tumor cells (CTCs) isolated from the blood, urine of the patients or other body fluids, exosomes, and circulating tumor nucleic acids, in particular suitable biological samples are blood samples, in particular whole blood samples.

In some embodiments, the gene expression levels of the signature genes may be integrated with other biomarkers to predict the progression of PCa. Suitable biomarkers for this purpose include, but are not limited to, germline and somatic mutations, DNA methylation markers, and protein markers. In some embodiments, the combination of the signature genes and other biomarkers can be implemented by including both the signature genes and the biomarkers in the same predictive model. In some embodiments, the effect of the other biomarkers may be accounted for in a computational mechanism in addition to the predictive model, such as a second model that combines the output of the first predictive model with the effects of the other biomarkers. One skilled in the art understands various approaches may be used to combine the effects of the signature genes and biomarkers to predict the progression of PCa.

In some embodiments, the gene expression levels of the signature genes may be measured multiple times. In some embodiments, the dynamics of the expression levels may be used in combination of the signature genes' expression levels to better predict the clinical outcome. One skilled in the art understands various approaches may be used to combine the effects of the levels and the dynamics of the signature genes' expression to predict the progression of PCa.

### Determining Gene Expression Level

The methods of the disclosure depend on the detection of differentially expressed genes for expression profiling across heterogeneous tissues. Thus, the methods depend on profiling genes whose expression in certain tissues is activated to a higher or lower level in an individual afflicted with a condition, for example, cancer, such as prostate cancer, relative to its expression in a non-cancerous biological sample or in a control subject. Gene expression can be activated to a higher or lower level at different stages of the same conditions and a differentially expressed gene can be either activated or inhibited at the nucleic acid level or protein level, or may be subject to alternative splicing to result in a different polypeptide product. Such differences can be evidenced by a change in mRNA levels, surface expression, secretion or other partitioning of a polypeptide, for example. For the purpose of this disclosure, differential gene expression is considered to be present when there is at least about 1.1-fold, 1.2-fold, 1.3-fold, 1.4-fold, 1.5-fold, 1.6-fold, 1.7-fold, 1.8-fold, 1.9-fold, to two-fold.

Differential signature gene expression can be identified or confirmed using methods known in the art such as qRT-PCR (quantitative reverse-transcription polymerase chain reaction) and microarray analysis. In particular embodiments, differential signature gene expression can be identified, or confirmed using microarray techniques.

### Practical Implementation in a Clinical Setting

In some embodiments, the above-described methods are integrated into a routine clinical laboratory workflow. For example, a blood sample from the patient may be collected into an RNA-stabilizing tube (e.g., PAXgene^{®}) and processed according to the manufacturer's instructions. After RNA extraction and quality assessment (with an RNA Integrity Number (RIN) of ≥7), cDNA is synthesized following standard protocols.

Gene expression levels of the 22 signature genes can then be measured using a standardized qPCR assay or a next-generation sequencing (NGS) platform. Appropriate normalization strategies, such as using one or more reference genes or global normalization methods, are employed to ensure accurate quantification. The normalized expression data are then input into the predictive model described herein, applying the previously determined model coefficients to generate a risk score.

Thresholds for clinical interpretation (e.g., distinguishing low-risk from high-risk patients) may be established using Receiver Operating Characteristic (ROC) curve analyses. A higher risk score correlates with an increased likelihood of disease progression, enabling clinicians to tailor treatment strategies-such as deciding whether to initiate, continue, or intensify therapies like enzalutamide-and to consider enrolling the patient in appropriate clinical trials. This systematic approach ensures consistent, reproducible integration of the gene signature into standard oncological care."

### Apparatus and Systems for Predicting Progression of PCa

[Analysis of the sequencing data and the diagnosis derived therefrom are typically performed using various computer executed algorithms and programs. Therefore, certain embodiments employ processes involving data stored in or transferred through one or more computer systems or other processing systems. Embodiments disclosed herein also relate to apparatus for performing these operations. This apparatus may be specially constructed for the required purposes, or it may be a general-purpose computer (or a group of computers) selectively activated or reconfigured by a computer program and/or data structure stored in the computer. In some embodiments, a group of processors performs some or all of the recited analytical operations collaboratively (e.g., via a network or cloud computing) and/or in parallel. A processor or group of processors for performing the methods described herein may be of various types including microcontrollers and microprocessors such as programmable devices (e.g., CPLDs and FPGAs) and non-programmable devices such as gate array ASICs or general purpose microprocessors.

In addition, certain embodiments relate to tangible and/or non- transitory computer readable media or computer program products that include program instructions and/or data (including data structures) for performing various computer-implemented operations. Examples of computer-readable media include, but are not limited to, semiconductor memory devices, magnetic media such as disk drives, magnetic tape, optical media such as CDs, magneto-optical media, and hardware devices that are specially configured to store and perform program instructions, such as read-only memory devices (ROM) and random access memory (RAM). The computer readable media may be directly controlled by an end user or the media may be indirectly controlled by the end user. Examples of directly controlled media include the media located at a user facility and/or media that are not shared with other entities. Examples of indirectly controlled media include media that is indirectly accessible to the user via an external network and/or via a service providing shared resources such as the "cloud." Examples of program instructions include both machine code, such as produced by a compiler, and files containing higher level code that may be executed by the computer using an interpreter.

In various embodiments, the data or information employed in the disclosed methods and apparatus is provided in an electronic format. Such data or information may include reads and tags derived from a nucleic acid sample, counts or densities of such tags that align with particular regions of a reference sequence (e.g., that align to a chromosome or chromosome segment), reference sequences (including reference sequences providing solely or primarily polymorphisms), counseling recommendations, diagnoses, and the like. As used herein, data or other information provided in electronic format is available for storage on a machine and transmission between machines. Conventionally, data in electronic format is provided digitally and may be stored as bits and/or bytes in various data structures, lists, databases, etc. The data may be embodied electronically, optically, etc.

In some embodiments, the disclosure provides a system for predicting progression of prostate cancer in an individual, the system comprising: an apparatus configured to determine expression levels of nucleic acids from a biological sample taken from the individual; and hardware logic designed or configured to perform operations comprising: (a) receiving expression levels of a collection of signature genes from a biological sample taken from said individual, wherein said collection of signature genes comprises at least two genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8; (b) applying the expression levels to a predictive model relating expression levels of said collection of signature genes with prostate cancer progression; and (c) evaluating an output of said predictive model to predict progression of prostate cancer in said individual. In some embodiments, said collection of signature genes comprises at least one gene selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8. In some embodiments, said collection of signature genes comprises at least two genes selected from the group consisting essentially of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8.

In some embodiments, the apparatus of the system includes a microarray. In some embodiments, the apparatus includes a next generation sequencer. In some embodiments, the apparatus includes a qPCR device.

### Sequencing Methods

In various embodiments, determination of gene expression levels may involve sequencing nucleic acids corresponding to genes of interests. Any of a number of sequencing technologies can be utilized.

Some sequencing technologies are available commercially, such as the sequencing-by-hybridization platform from Affymetrix Inc. (Sunnyvale, CA) and the sequencing-by-synthesis platforms from 454 Life Sciences (Bradford, CT), Illumina/Solexa (Hayward, CA) and Helicos Biosciences (Cambridge, MA), and the sequencing-by-ligation platform from Applied Biosystems (Foster City, CA), as described below. In addition to the single molecule sequencing performed using sequencing-by-synthesis of Helicos Biosciences, other single molecule sequencing technologies include, but are not limited to, the SMRT^{™} technology of Pacific Biosciences, the ION TORRENTTM technology, and nanopore sequencing developed for example, by Oxford Nanopore Technologies.

It is understood that modifications which do not substantially affect the activity of the various embodiments of this disclosure are also included within the definition of the disclosure provided herein. Accordingly, the following examples are intended to illustrate but not limit the present disclosure.

### Examples

### MATERIAL AND METHODS

### Study design and conduct

The PREMIERE trial, a translational multicenter single-arm open-label phase 2 clinical trial (NCT02288936), investigated the use of enzalutamide as first-line treatment for metastatic castration-resistant prostate cancer (mCRPC). The study was approved by a central independent review board (IRB).

The chosen patients for the trial had histologically confirmed adenocarcinoma of the prostate with documented metastases and tumor progression, with a serum testosterone level of 50 ng per deciliter or less while continuing androgen-deprivation therapy. They had an Eastern Cooperative Oncology Group (ECOG) 0-1 and were asymptomatic or mildly symptomatic (Brief Pain Inventory Short Form question 3 of less than 4).

The validation cohort consisted of a single institution cohort from patients participating in a protocol approved by the Istituto Scientifico Romagnolo per Io Studio e la Cura dei Tumori (IRST), Meldola, Italy (REC 2192/2013) with plasma samples collected prospectively with the primary aim of biomarker evaluation.

### Patients

The PREMIERE trial included 98 chemo-naïve mCRPC patients from 17 hospitals in Spain treated at a dose of 160 mg as first-line treatment with enzalutamide. Whole-blood samples were serially obtained at pre-treatment, at 12 weeks during the treatment, and at tumour progression.

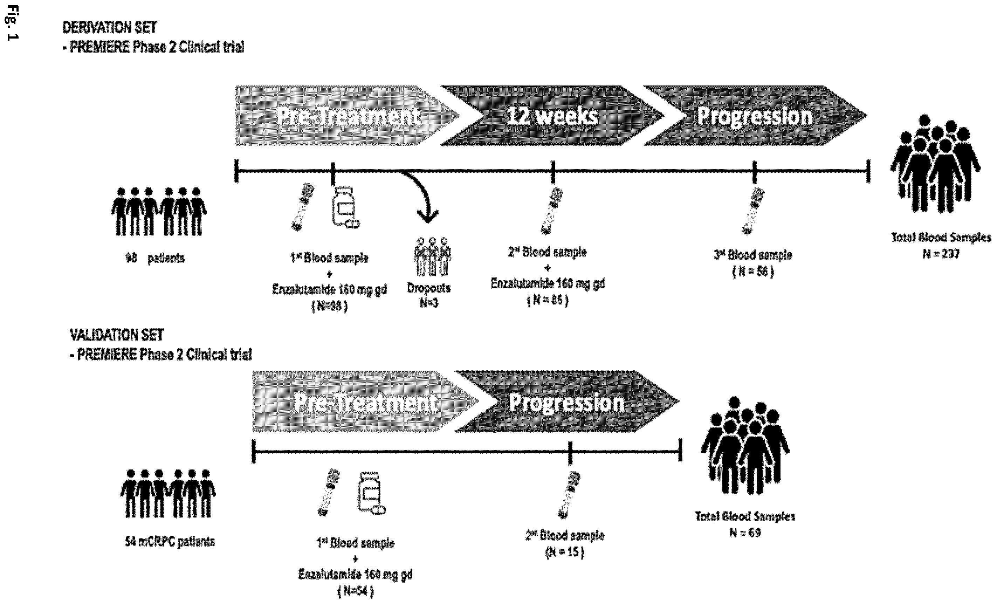

A total of 237 whole-blood samples were prospectively collected. After quality controls, 3 blood samples were excluded due to poor RNA integrity **Figure 1****.**

In the IRST trial, whole-blood samples were obtained at pre-treatment and at tumour progression. A total of 69 whole blood samples were collected from 54 patients.

### RNA extraction and microarray analysis

Whole-blood samples were collected in whole-blood PAXgene RNA, isolated and purified as established by the manufacturer's instructions. RNA quantification and quality assessment were performed employing spectrophotometry and electrophoresis on microfluidic solution with NanoDrop 2000 (Thermo Scientific, Newark, DE, USA) and Bioanalyzer 2100 (Agilent Technologies, Palo Alto, CA, USA), respectively. Only purified RNA samples with an RNA integration number (RIN) of seven or more were selected for further analyses. 100 ng of RNA of each sample was used to synthetize complementary DNA (cDNA) with the WT PLUS Reagent Kit (Thermo Scientific, Newark, DE, USA) following the standard protocol. The cDNA was amplified, fragmented, and labelled with biotine for the hidridization process.

Microarrays GeneChipTM Human Trascriptome Array HTA 2.0 (902162, Affymetrix, ThermoFisher, Newark, DE, USA) were hybridized using 5.2 ug of single strand DNA (ssDNA) during 16 hours, washed to eliminate the unspecific probes and then scanned with GeneChip Scanner 3000 (Affymetrix, Thermo Scientific, Newark, DE, USA).

### Differential expression analysis and identification of DEGs

Gene microarray analyses were derived after a quality control, background correction, normalization, logarithmic conversion and remove batch effects processing using Transcriptome Analysis Console (TAC) Software (version 4.0.1, Thermo). All samples that had not passed quality control were filtered out and the resulting data were annotated and analysed by R packages "affy", "limma" and "pd.hta.2.0"²⁴⁻²⁶. The P-value <0,01, a Q-value <0,05 and | log2FC|>0.3 were set as the threshold for screening DEGs.

### Clinical Data Processing

Clinical data was initially loaded into a data frame. Preprocessing steps included converting survival time from months to days and reformating variable names for consistency. Differential expression analysis was conducted to filter pre-treatment gene expression values, focusing on genes significantly associated with the patients' conditions.

### Survival Analysis Methods

### Univariable Analysis

Univariable Cox regression analyses were performed for each gene using a parallel computing approach (RegParallel) to efficiently handle the high-dimensional data. Genes significantly influencing survival outcomes were identified, with p-values adjusted for multiple testing using the false discovery rate method. This step was crucial in reducing the number of genes to be considered for further analysis.

### Model Training

Following the univariable analysis, several advanced survival models were developed to analyze the data, using the selected genes:
1. **Cox Proportional Hazards model with Ridge regularization:**
   ∘ A Ridge regression version of the Cox model was implemented, where regularization parameters were optimized through grid search. This method was employed to control for overfitting and enhance the model's generalizability.
2. **LASSO-penalized Cox model:**
   ∘ The LASSO technique was applied to perform variable selection, effectively shrinking the coefficients of less critical variables to zero. This step helped in reducing model complexity and focusing on the most predictive features.
3. **Elastic Net-penalized Cox model:**
   ∘ Combined both Ridge and LASSO penalties to optimize model complexity and variable selection. This model aimed to balance feature selection with model stability, taking advantage of the benefits of both regularization methods.
4. **Random Survival Forest:**
   ∘ A Random Survival Forest model was trained to manage non-linear relationships between variables and survival times. Feature importance was evaluated through permutation importance tests to identify significant predictors, providing insights into the variables most impacting survival outcomes.

### Grid Search and Cross-Validation

For each model, hyperparameters were fine-tuned using GridSearchCV with a K-Fold cross-validation strategy. This method ensured that each model was not only generalizable across different subsets of the data but also robust against variations within the training data.

### Feature Selection and Model CoefFicients

For each regression-based model, coefficients were examined to determine the influence of each variable on survival predictions. This analysis was essential for identifying key features that significantly impact the survival outcomes, informing further feature selection to refine the prognostic signature.

### Validation and Prediction

The optimized models were then applied to a separate validation dataset to assess their predictive performance. This step was crucial for confirming the models' effectiveness in an independent cohort, thereby reinforcing the reliability and applicability of the prognostic signature in clinical settings.

### Model Evaluation and Results Visualization

Model performances were visualized using plots of the concordance index (c-index) across different hyperparameter values. Additionally, the coefficients of significant predictors were plotted to highlight their relative importance and impact on the survival outcomes. The final evaluation included a comprehensive comparison of the models' performance on the validation dataset, summarized in a results table displaying the c-index for each model. Finally, LASSO model was selected.

### Combinatorial Testing of Top Features

The top features identified by the LASSO model were further tested in combinations to evaluate their collective impact on model performance. This step was crucial for refining the prognostic signature and determining the most predictive subset of features.

### Validation and Prediction

### Independent Validation

The optimized LASSO model was applied to a separate validation dataset to confirm its effectiveness in an independent cohort. This step assessed the model's predictive performance and robustness.

### Prediction and Final Evaluation

The final evaluation of the model included predictions on the validation dataset and a comprehensive comparison of model performance, summarized in a results table displaying the c-index for both training and validation datasets, obtaining a model of 22 genes:

### coefficient

| | |
|---|---|
| **SNORD88C** | 0.517364 |
| **SYT14L** | 0.469121 |
| **MS4A2** | -0.310436 |
| **AC092755.4** | 0.290974 |

### coefficient

| | |
|---|---|
| MAP2K4 | 0.290191 |
| CXADRP2 | 0.287535 |
| MS4A6A | 0.235089 |
| SLC31A1 | 0.231427 |
| IL5RA | -0.224362 |
| RNU6.286P | 0.178509 |
| MIR142 | -0.168221 |
| SRGAP2B | 0.131916 |
| ADAM17 | 0.129305 |
| IFI27 | 0.128270 |
| CLC | -0.120376 |
| RELL1 | 0.119308 |
| TSPAN2 | 0.100680 |
| NUDT4 | 0.058931 |
| MIR4503 | -0.039713 |

### coefficient

| | |
|---|---|
| PTBP3 | 0.021022 |
| NFKBIA | 0.011279 |
| MMP8 | 0.007688 |

With the following C-index:

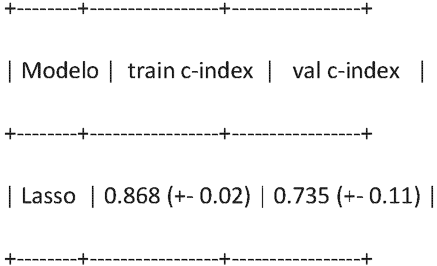

This structured approach to developing and validating the LASSO model ensures a rigorous assessment of its predictive capabilities and emphasizes its potential for clinical application in predicting patient outcomes based on survival data.

## Claims

1. A method for predicting progression of prostate cancer in an individual, the method comprising:
(a) receiving expression levels of a collection of signature genes from a biological sample taken from said individual, wherein said collection of signature genes comprises at least 2, 3, 4, 5, 6, 7, 8, 9 ... 22 genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8;
(b) applying the expression levels to a predictive model relating expression levels of said collection of signature genes with prostate cancer progression; and
(c) evaluating an output of said predictive model to predict progression of prostate cancer in said individual.

2. The method for predicting progression of prostate cancer in an individual according to claim 1, the method comprising:
(a) receiving expression levels of a collection of signature genes from a biological sample taken from said individual, wherein said collection of signature genes comprises at least all genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8, and
(b) applying the expression levels to a predictive model relating expression levels of said collection of signature genes with prostate cancer progression; and
(c) evaluating an output of said predictive model to predict progression of prostate cancer in said individual.

3. The method according to any one of claims 1 to 2, wherein the prostate cancer is metastatic castration-resistant prostate cancer (mCRPC).

4. The method of any of the preceding claims, wherein said output of the predictive model predicts a likelihood of clinical recurrence of prostate cancer in the individual after said individual has undergone treatment for prostate cancer, wherein said treatment is enzalutamide.

5. The method according to claim 4, wherein said output of the predictive model predicts a likelihood of biochemical recurrence of prostate cancer in the individual after said individual has undergone treatment for prostate cancer.

6. The method according to claim 4, wherein said output of the predictive model predicts a likelihood of overall survival of the individual after said individual has undergone treatment for prostate cancer.

7. The method of any of the preceding claims, further comprising combining the gene expression levels of said signature genes with one or more other biomarkers to predict progression of prostate cancer in said individual such as one or more other biomarkers selected from the group consisting of germline mutations, somatic mutations, DNA methylation markers, protein markers, and any combinations thereof.

8. The method of any of the preceding claims, wherein the expression levels of a collection of signature genes comprise gene expression levels measured at multiple times.

9. The method of any of the precent claims, wherein the biological sample is a biological fluid selected from the group consisting of blood, preferably whole blood.

10. A system for predicting progression of prostate cancer in an individual, the system comprising:
an apparatus configured to determine expression levels of nucleic acids from a biological sample taken from the individual; and
hardware logic designed or configured to perform operations comprising:
(a) receiving expression levels of a collection of signature genes from a biological sample taken from said individual, wherein said collection of signature genes comprises at least two genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8;
(b) applying the expression levels to a predictive model relating expression levels of said collection of signature genes with prostate cancer progression; and
(c) evaluating an output of said predictive model to predict progression of prostate cancer in said individual.

11. The system for predicting progression of prostate cancer in an individual according to claim 10, wherein said collection of signature genes comprises at least all genes selected from the group consisting of: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8; wherein the prostate cancer is metastatic castration-resistant prostate cancer (mCRPC), and wherein said output of the predictive model predicts a likelihood of clinical recurrence of prostate cancer in the individual after said individual has undergone treatment for prostate cancer, wherein said treatment is enzalutamide.

12. The system according to any one of claims 10 or 11, wherein the biological sample is a body fluid selected from the group consisting of blood, preferably whole blood.

13. A method comprising:
- determining a gene expression level for the signature genes: SNORD88C, SYT14L, MS4A2, AC092755.4, MAP2K4, CXADRP2, MS4A6A, SLC31A1, IL5RA, RNU6.286P, MIR142, SRGAP2B, ADAM17, IFI27, CLC, RELL1, TSPAN2, NUDT4, MIR4503, PTBP3, NFKBIA and MMP8, to obtain a subject expression profile for a subject,
and further comprising:
- classifying the subject as having a good prognosis or a poor prognosis of prostate cancer based on the subject expression profile, wherein the good prognosis predicts an increased likelihood of survival within a predetermined period after initial diagnosis and/or no progression of disease after primary treatment, and the poor prognosis predicts an aggressive disease, a decreased likelihood of survival, and an increased likelihood of biochemical recurrence, clinical recurrence, and/or the presence of local or distant metastases, within a predetermined period after initial diagnosis; and/or
- classifying the subject as having or not having a predisposition of prostate cancer that is susceptible to disease progression based on the subject expression profile, wherein the predisposition predicts an aggressive disease, decreased likelihood of survival, and an increased likelihood of biochemical recurrence, clinical recurrence, and/or the presence of local or distant metastases, within a predetermined period after initial diagnosis.

14. The method according to claim 13, wherein the prostate cancer is metastatic castration-resistant prostate cancer (mCRPC), and wherein said individual has undergone treatment for prostate cancer, and wherein said treatment is enzalutamide.

15. A composition comprising enzalutamide for use in the treatment of metastatic castration-resistant prostate cancer (mCRPC) in an individual having said disease and classified as having good prognosis in accordance with any one of claims 13 or 14.
